Europäisches Patentamt

European Patent Office

Office européen des brevets

(11) Veröffentlichungsnummer : **0 346 600 B1**

## (12) EUROPÄISCHE PATENTSCHRIFT

(45) Veröffentlichungstag der Patentschrift :
**01.04.92 Patentblatt 92/14**

(21) Anmeldenummer : **89107320.7**

(22) Anmeldetag : **22.04.89**

(51) Int. Cl.⁵ : **A61K 6/04, C22C 5/00**

(54) **Anlaufbeständige Edelmetalllegierungen für die Zahntechnik.**

(30) Priorität : **11.06.88 DE 3819904**

(43) Veröffentlichungstag der Anmeldung :
**20.12.89 Patentblatt 89/51**

(45) Bekanntmachung des Hinweises auf die
Patenterteilung :
**01.04.92 Patentblatt 92/14**

(84) Benannte Vertragsstaaten :
**AT BE CH DE ES FR GB GR IT LI LU NL SE**

(56) Entgegenhaltungen :
**EP-A- 0 057 149**
**FR-A- 2 291 282**
**FR-A- 2 576 321**
**FR-A- 2 620 133**

(73) Patentinhaber : **Degussa Aktiengesellschaft**
**Weissfrauenstrasse 9**
**W-6000 Frankfurt am Main 1 (DE)**

(72) Erfinder : **Kropp, Rudolf, Dr. Dipl.-Chem.**
**Hölderlinstrasse 2**
**W-7530 Pforzheim (DE)**
Erfinder : **Kürten, Wolfgang**
**Karl-Strasse 8**
**W-7534 Birkenfeld (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 346 600 B1

EP 0 346 600 B1

## Beschreibung

Die Erfindung betrifft anlaufbeständige Edelmetalllegierungen auf Basis Silber-Gold-Palladium-Kupfer mit Zusätzen von Indium und Zink für die Zahntechnik, insbesondere zum Gießen von Kronen und Brücken, die mit Kunststoff verblendet werden sollen oder auch unverblendet bleiben.

Legierungen mit mindestens 75 % Gold und Platinmetallen haben sich seit Jahrzehnten in der Zahntechnik bestens bewährt, sind jedoch sehr teuer. Auch Legierungen mit 50-60 % Gold und Zusätzen von 5-10% Palladium sind seit vielen Jahren bekannt, aber ebenfalls noch relativ teuer.

Deutlich preisgünstigere Edelmetallegierungen mit Goldgehalten unter 50 %, Palladiumgehalten von 5-30 %, mit hohem Silbergehalt und Zusätzen an Indium, Kupfer und Zink sind ebenfalls bekannt, unterscheiden sich jedoch von den höher goldhaltigen Legierungen dadurch, daß sie normalerweise ein zweiphasiges Gefüge aufweisen.

Diese zweite Phase ist stets mit Palladium und Indium stark angereichert, was zu Abreicherung dieser Elemente in der silberreichen Matrix führt. Die Anlaufbeständigkeit von silberreichen Legierungen hängt aber in starkem Maße von ihrem Gehalt an Palladium un Indium ab, die beide das Anlaufen der Oberfläche, durch Sulfide zurückdrängen.

Die Zweiphasigkeit des Gefüges dieser Legierungen ist weiterhin mit dem Nachteil verbunden, daß ihr Korrosionspotential gegenüber unterschiedlichen Anionen und Komplexbildnern stark differieren kann, so daß es zu Lokalelementbildungen kommt und dadurch zu einer verstärkten Korrosion. Da die palladiumreiche Phase härter ist als die silberreiche, kann es außerdem bei der Politur einer Krone oder Brücke leicht zu einer ungleichmäßigen Oberfläche kommen (Orangenhaut).

Ein weiterer Nachteil ist die verminderte Verformbarkeit (Dehnung) von zweiphasigen Legierungen.

Es war daher Aufgabe der vorliegenden Erfindung, anlaufbeständige Edelmetallegierungen auf der Basis Silber-Gold-Palladium-Kupfer mit Zusätzen an Indium und Zink für die Zahntechnik zu entwickeln, insbesondere zum Gießen von Kronen und Brücken, die preisgünstig, einphasig und korrosionsfest sind und außerdem eine hohe Härte und gute Verformbarkeit aufweisen.

Diese Aufgabe wird erfindungsgemäß dadurch gelöst, daß sie aus 36 bis 39 Gewichts% Silber, 28 bis 32,5 Gewichts% Gold, 17 bis 19,5 Gewichts% Palladium, 0 bis 0,1 Gewichts% Iridium, 9,5 bis 10,5 Gewichts% Kupfer, 0,5 bis 3 Gewichts% Indium und 0,5 bis 2,5 Gewichts% Zink bestehen, wobei die Summe von Indium und Zink maximal 4 Gewichts% betragen darf.

Vorzugsweise enthalten die Legierungen 37 bis 38 Gewichts% Silber, 30 Gewichts% Gold, 18 bis 19 Gewicht% Palladium, 0 bis 0, 1 Gewichts % Iridium, 10 Gewichts% Kupfer, 1 ,5 bis 2,5 Gewichts% Indium und 1 bis 1 ,5 Gewichts% Zink.

Überraschenderweise hat es sich gezeigt, daß es bei Legierungen auf Basis Silber-Gold-Palladium-Kupfer-Indium-Zink mit etwa 30% Gold einen relativ schmalen Bereich von Legierungzusammensetzungen gibt, die sowohl eine hohe Härte ,Dehngerenze und Zugfestigkeit besitzen, die sie schon nach langsamer Abkühlung eines Gusses in der Gußform erreichen, als auch eine gute plastische Dehnung aufweisen, darüber hinaus vor allem einphasig sind und damit die gewünschte Voraussetzung für eine gute Anlauf- und Korrosionsbeständigkeit bieten. Legierungen, die diese genannten Voraussetzungen erfüllen, haben Massen-Gehalte von 36-39 % Silber, 28-32,5 % Gold, 17 - 19,5 % Palladium, 0-0,1 % Iridium, 9,5-10,5 % Kupfer, 0,5-3,0 % Indium und 0,5-2,5 % Zink, wobei die Summe von Indium und Zink maximal 4 % betragen darf.

In der folgenden Tabelle sind 13 Legierungen angegeben, denen die Legierungen 9-13 außerhalb der erfindungsgemäßen Zusammensetzung liegen und durchweg ein zweiphasiges Gefüge aufweisen, bei dem eine Palladium-Indium-Zink-reiche Phase in eine Gold-Silber-reiche Matrix-Phase eingebettet ist. Diese letztere Phase ist gegenüber bestimmten Anionen, die im Speichel vorkommen, wie z.B. Rhodanid und Nitrit, korrosionschemisch unedler als die palladiumreiche Phase und kann daher bevorzugt im Munde angegriffen werden. Die Legierungen 10-12 sind trotz nur geringer Überschreitung der erfindungsgemäßen Zusammensetzung bereits zweisphasig und zeigen nach dem Guß Härtewerte von deutlich unter 200 HV.

Die Legierungen 1-8 sind nach dem Guß einphasig, erreichen Härtewerte von mindestens 230 HV, 0,2-%-Dehngrenzen von mindestens 660 N/mm$^2$, Zugfestigkeiten von mindestens 795 N/mm$^2$ und Bruchdehnungen von mindestens 10 %. Sie sind daher den bekannten zweiphasigen Legierungen deutlich überlegen.

2

Tabelle

| Legierung Nr. | Zusammensetzung in Massen-% | | | | | | | Schmelz-intervall °C | Zahl der Phasen | technische Daten, Guß langsam abgekühlt | | | |
|---|---|---|---|---|---|---|---|---|---|---|---|---|---|
| | Au | Pd | Ir | Ag | Cu | In | Zn | | | Härte HV | 0,2-%-Dehngrenze N/mm² | Zug-festigk. N/mm² | Bruch-dehnung % |
| 1 | 29,0 | 18,9 | 0,1 | 38,0 | 10,0 | 2,5 | 1,5 | 1015- 905 | 1 | 245 | 730 | 850 | 13 |
| 2 | 31,0 | 18,9 | 0,1 | 36,5 | 10,0 | 2,5 | 1,0 | 1020- 930 | 1 | 255 | 810 | 910 | 11 |
| 3 | 28,0 | 19,4 | 0,1 | 38,5 | 10,5 | 2,0 | 1,5 | 1020- 930 | 1 | 230 | 720 | 835 | 12 |
| 4 | 30,0 | 18,9 | 0,1 | 38,0 | 10,0 | 2,0 | 1,0 | 1030- 935 | 1 | 260 | 775 | 880 | 12 |
| 5 | 30,0 | 18,9 | 0,1 | 37,5 | 10,0 | 2,0 | 1,5 | 1015- 925 | 1 | 255 | 725 | 845 | 10 |
| 6 | 30,0 | 18,9 | 0,1 | 37,5 | 10,0 | 2,5 | 1,0 | 1025- 920 | 1 | 260 | 765 | 861 | 12 |
| 7 | 30,0 | 18,9 | 0,1 | 38,0 | 10,0 | 1,5 | 1,5 | 1020- 935 | 1 | 255 | 725 | 855 | 15 |
| 8 | 32,0 | 17,5 | - | 37,0 | 9,5 | 1,5 | 2,5 | 995- 910 | 1 | 230 | 660 | 795 | 10 |
| 9 | 20,0 | 21,0 | 0,1 | 39,0 | - | 16,0 | 4,0 | 1035- 860 | 2 | 155 | 280 | 565 | 8 |
| 10 | 28,0 | 17,9 | 0,1 | 40,5 | 9,5 | 0,5 | 3,5 | 985- 890 | 2 | 160 | | | |
| 11 | 30,0 | 17,9 | 0,1 | 39,0 | 9,5 | 0,5 | 3,0 | 990- 900 | 2 | 170 | | | |
| 12 | 32,5 | 17,0 | 0,1 | 37,0 | 9,5 | 1,0 | 3,0 | 980- 920 | 2 | 185 | | | |
| 13 | 40,0 | 7,9 | 0,1 | 35,0 | 7,0 | 5,0 | 3,5 | 850- 770 | 2 | 245 | 650 | 780 | 7 |

EP 0 346 600 B1

3

**Patentansprüche**

1. Anlaufbeständige Edelmetallegierungen auf Basis Silber-Gold-Palladium-Kupfer mit Zusätzen von Indium und Zink für die Zahntechnik, insbesondere zum Gießen von Kronen und Brücken, dadurch gekennzeichnet, daß sie aus 36-39 Gewichts% Silber, 28,0-32,5 Gewichts% Gold, 17,0-19,5 Gewichts% Palladium, 0-0,1 Gewichts% Iridium, 9,5-10,5 Gewichts% Kupfer, 0,5-3,0 Gewichts% Indium und 0,5-2,5 Gewichts% Zink bestehen, wobei die Summe von Indium und Zink maximal 4 Gewichts% betragen darf.

2. Anlaufbeständige Edelmetallegierungen nach Anspruch 1, dadurch gekennzeichnet, daß sie 37-38 Gewichts% Silber, 30 Gewichts% Gold, 18-19 Gewichts% Palladium, 0-0,1 Gewichts% Iridium, 10 Gewichts% Kupfer, 1,5-2,5 Gewichts% Indium und 1,0-1,5 Gewichts% Zink enthalten.

**Claims**

1. Tarnish resistant noble metal alloys based on silver-gold-palladium-copper with additions of indium and zinc for dentistry, in particular for casting crowns and bridges, characterised in that they consist of from 36 to 39% by weight of silver, from 28.0 to 32.5% by weight of gold, from 17.0 to 19.5% by weight of palladium, from 0 to 0.1% by weight of iridium, from 9.5 to 10.5% by weight of copper, from 0.5 to 3.0% by weight of indium and from 0.5 to 2.5% by weight of zinc, and the sum of indium and zinc must not exceed a maximum of 4% by weight.

2. Tarnish resistant noble metal alloys according to Claim 1, characterised in that they contain from 37 to 38% by weight of silver, 30% by weight of gold, from 18 to 19% by weight of palladium, from 0 to 0.1% by weight of iridium, 10% by weight of copper, from 1.5 to 2.5% by weight of indium and from 1.0 to 1.5% by weight of zinc.

**Revendications**

1. Alliages de métaux nobles à base d'argent-or-palladium-cuivre avec additions d'indium et de zinc, résistants au ternissement, pour la technique dentaire, en particulier pour la coulée de couronnes et de bridges, caractérisés en ce qu'ils sont constitués de 36 à 39 % en poids d'argent, de 28 à 32,5 % en poids d'or, de 17,0 à 19,5 % en poids de palladium, de 0 à 0,1 % en poids d'iridium, de 9,5 à 10,5 % en poids de cuivre, de 0,5 à 3,0 % en poids d'indium et de 0,5 à 2,5 % en poids de zinc, la somme de l'indium et du zinc devant être au maximum de 4 % en poids.

2. Alliages de métaux nobles résistants au ternissement selon la revendication 1, caractérisés en ce qu'ils contiennent de 37 à 38 % en poids d'argent, 30 % en poids d'or, de 18 à 19 % en poids de palladium, de 0 à 0,1 % en poids d'iridium, 10 % en poids de cuivre, de 1,5 à 2,5 % en poids d'indium et de 1,0 à 1,5 % en poids de zinc.